# EUROPEAN PATENT APPLICATION

(11) **EP 1 230 841 A1**
(43) Date of publication of application: **14.08.2002**
(21) Application number: 01102959.2
(22) Date of filing: 08.02.2001
(51) Int. Cl.: A01H 3/02, A01H 3/04

(54) **Control of campanula habit**

(71) Applicant: GARTNERIET PKM ApS, 5270 Odense N (DK)
(72) Inventor: Jensen, Gert Kim, 5270 Odense N (DK)
(74) Representative: Roerboel, Leif

(57) **Abstract**

The present invention relates to a method of controlling the habit of Campanula (1). By treating the plants with 2-chloroethyiphosphonic acid at the end of the vegetative growth period, the habit becomes upright and more branched.

## Description

### TECHNICAL FIELD

The present invention relates to a method of controlling the habit of Campanula as set forth in the preamble of claim 1.

### BACKGROUND ART

Campanula are popular pot plants. Industrial production of Campanula for sales in pots takes largely place in greenhouses. The operation of greenhouses is expensive compared to outdoor operation and therefore the optimum use of space and time is crucial. The plants are ready for sale when they have sufficient body and also flower. It is therefore crucial to bring the plants to a sales-ready state as fast as possible.

Some of the species in the Campanula genus have low and spreading habits and the stems have a relatively strong apical dominance, i.e. the apical meristem is able to produce hormones to prevent side shoots or buds from developing while it is growing. Therefore these type of Campanula require a long time (in the greenhouse) until they reach a habit and size that gives sufficient body and an attractive bouquet of flowers that is suitable for sales.

Ethylene is a gaseous plant hormone, which is involved in numerous aspects of plant growth and development from seed germination through senescence and death of the plant.

It is well known that ethylene can cause the premature death of plants including flowers, leaves, fruits and vegetables. It can also promote leaf yellowing and stunted growth as well as premature fruit, flower and leaf drop.

It is also used in the acceleration of ripening of fruits and vegetables

### DISCLOSURE OF THE INVENTION

On this background, it is the object of the present invention to provide a method of controlling the habit of Campanula of the kind referred to initially, which overcomes the above-mentioned problems. This object is achieved in accordance with claim 1 by applying ethephon towards the end of the short-day light conditions. The habit becomes more upright and the stems loose their apical dominance and side shoots develop at an early stage resulting in more flowers. The plants reach a state in which they are ready for sales earlier resulting in a shorter overall stay in the relatively expensive greenhouse. Further, it was observed that the lower side shoots grow more upright, thus further increasing the attractiveness of the habit and allowing the plants to be placed closer to one another in the greenhouse.

According to an embodiment of the invention, the period of short-day light conditions comprises a winter simulation sub-period with substantially complete darkness and low temperatures. The plants react by generative growth and flowering in the warmer long-day light conditions following the simulated winter.

According to another embodiment of the invention, the ethephon is applied as a solution with a concentration of 0.2 to 5 ‰, preferably as a spray.

### BRIEF DESCRIPTION OF THE DRAWINGS

In the following detailed portion of the present description, the invention will be explained in more detail with reference to the exemplary embodiments shown in the drawings, in which
Figure 1 shows a Campanula Portenschlagiana grown according to prior art methods, and
Figure 2 shows a Campanula Portenschlagiana with a habit controlled in accordance with the invention.

### DETAILED DESCRIPTION OF THE INVENTION

Plant growth responses are affected by both internal and external factors. Internal control of plant processes is under the influence of genetic expression of the biological clocks of the plant. These processes influence both the extent and timing of growth processes. Such responses are mediated by signals of various types, which are transmitted within and between cells. Intracellular communication in plants typically occurs via hormones (or chemical messengers) as well as other less understood processes.

Because communications in a plant are typically mediated by plant hormones, both the presence and levels of such hormones are important to specific plant cell reactions. The plant hormone that is most relevant to the present invention is ethylene, which has the capacity to affect many important aspects of plant growth, development and senescence. The most important effects of ethylene include processes normally associated with senescence

The genus Campanula comprises about 300 species of annuals, biennials and perennials. Most of these species are easily cultivated and provide a display of long lasting flowers. Leaves are entire or toothed and borne alternately. Flowers are borne in panicles, racemes or clustered heads. Occasionally, they are solitary. Their shape varies from tubular to bell- or star-shaped. They may also be more open and be cup- or saucer-shaped. Propagation for all but double-flowered forms is easy from seed, although some are slow to germinate and develop (e.g. Campanula Formanekiana). Seed may be sown in containers and placed in a cold frame in the spring. Plants range from dwarf mat forming to those that form clumps or trail to tall-growing upright species. This invention pertains to those species of Campanula that display an apical dominance during their development. Typical species in this group are Campanula Portenschlagiana (Dalmation Bellflower), Campanula Elationoides, Campanula Garganica, and Campanula Fenestralate.

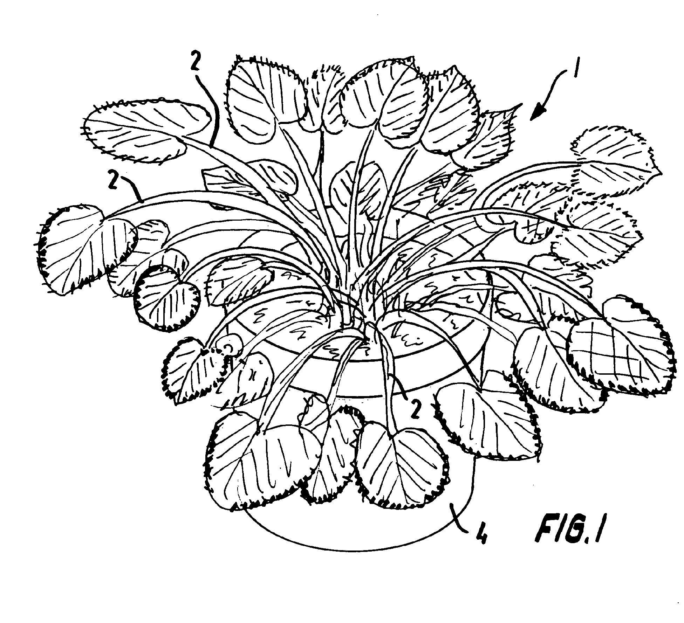

Fig. 1 shows a Campanula Portenschlagiana 1 which has reached a mature size allowing the vegetative growth stage to be ended and the generative growth to be induced. The habit is low and spreading and a considerable part of the stems 2 extends substantially horizontal or at a sharp angle with the horizontal. The stems 2 have few side-shoots 3 because of the apical dominance of the meristem. When the generative growth is induced, a considerable part of the flowers develop from the lower substantially horizontally extending stems 2. This distribution of the flowers and the low habit is the reason that it is often grown there where it can hang, such as in a basket arrangement. For industrial production of these plants in pots 4 in greenhouses and for attractive appearance on the shelves for sales a hanging arrangement is impractical (handling) and space consuming. And a low habit is not considered as attractive and is considered a commercial disadvantage. An upright habit such as naturally displayed by e.g. Campanula Carpatica would therefore be advantageous from a practical and marketing point of view.

According to a preferred embodiment of the invention, Campanula 1 are grown from plantlets (not shown) that are obtained from seedlings or cuttings. The plantlets are grown to Campanula plants 1 of a more mature size. The first weeks (6 to 10 weeks, preferably 8 weeks) of the vegetative growth are carried out under short-day light conditions, i.e. with 6 to 14 hours light. The number of weeks in short daylight and the hours of daylight depend on the type of Campanula and other conditions.

The next 3 to 8 weeks, preferably 5 weeks, of the vegetative growth is carried out under short daylight and cold (+2 to +12° C) conditions. This is a winter simulation sub-period provided at the end of the short-day period.

The short-day growing period lasts for 8 to 16 weeks depending on the type of Campanula, and other conditions, i.e. greenhouse operation or not, soil and fertiliser, CO2 treatment.

Towards the end of the short-day light conditions period, 2-chloroethylphosphonic acid is applied to the plants. According to a preferred embodiment, the 2-chloroethylphosphonic acid is applied 3 to 4 days before the end of the short-day light conditions period.

The formula for 2-chloroethylphosphonic acid is C₂H₆ClO₃P and it is identified with the short name ethephone. Trade names for products containing ethephon include Arvest, Bromeflor, Etheverse, Flordimex, Flordimex T-Extra, Cerone, Etherel, Chipco Florel Pro and Prep and the main manufacturer is Rhone-Poulenc.

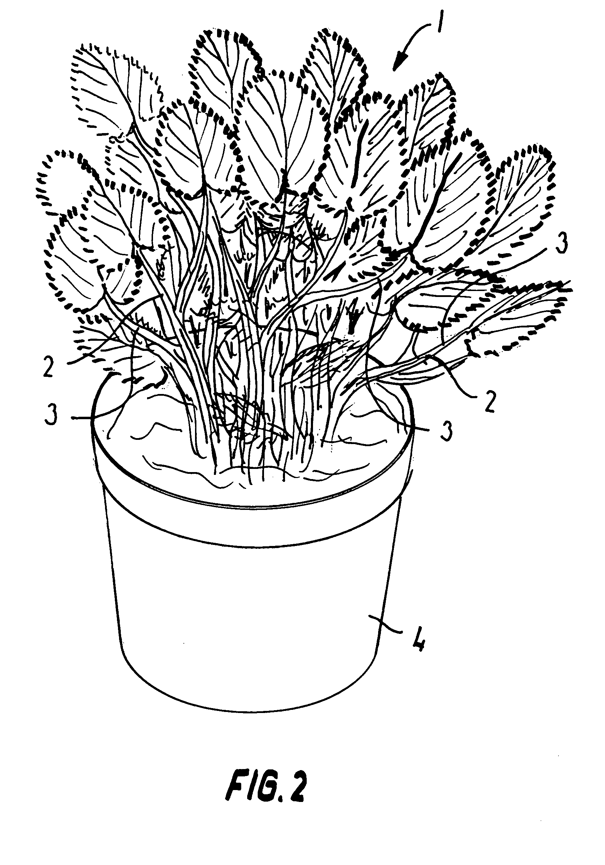

2-chloroethylphosphonic acid's mode of action acts via liberation of ethylene, an important component of the plant hormone complex. The ethylene is absorbed by the plant and interferes in the growth process. After the treatment, the stems 2 grow more upright and there appear more side shoots 3. Consequently, the overall habit of the plant becomes more upright and after the side shoots 3 develop, there are more branches, in particular on the top of the plant. The effect of the treatment is shown in figure 2. Shortly after the treatment with 2-chloroethylphosphonic acid the plants are exposed to long-day light conditions to induce generative growth.

It is well known that ethylene can cause the premature death of plants including flowers, leaves, fruits and vegetables. It can also promote leaf yellowing and stunted growth as well as premature fruit, flower and leaf drop. Wrong timing of the treatment with 2-chloroethylphosphonic acid would therefore have catastrophic consequences. According to a preferred embodiment, the long-day light condition period is started a week after the treatment with 2-chloroethylphosphonic acid. According to another preferred embodiment, the long-day light condition period is started three to four days after the treatment with 2-chloroethylphosphonic acid. During the period of long-day light conditions, the plants are exposed to 12 to 24 hours of daylight. The long exposure to light induces the generative growth, i.e. the plant starts to develop flowers. The long-day light period lasts for 5 to 12 weeks, depending on conditions such as type of Campanula, temperature and humidity, intensity of the light soil and fertiliser etc.

When the flowers have developed sufficiently, the plants are ready for sale. Because of the changed habit the plants according to the present invention have more flowers which are located on the top of the plant, thus giving the plants a more attractive appearance. Because of the changed habit it is possible to start the generative growth earlier and obtain an attractive and mature appearing sales plant with abundant flowers. Therefore the plants remain in the greenhouse for a shorter period, thus improving the economic aspects of the production of the plants.

### LIST OF REFERENCE NUMERALS

- 1.: Campanula
- 2.: Stem
- 3.: Side shoot
- 4.: Pot

## Claims

1. Method of controlling the habit of Campanula (1) comprising the steps of:
- providing Campanula plantlets,
- providing a period of short-day light conditions to stimulate vegetative growth during least a part of a time span in which the Campanula (1) develop from plantlets to plants,
- applying 2-chloroethylphosphonic acid to said plants towards the end of said period of short-day light conditions, and
- switching from short-day light conditions to long-day light within 7 days after applying 2-chloroethylphosphonic acid to stimulate generative growth.

2. Method according to claim 1, wherein said period of short-day light conditions comprises a winter simulation sub-period in short-day light conditions and low temperatures.

3. Method according to claim 1 or 2, wherein said period of short-day light spans 6 to 16 weeks, preferably 8 to 12 weeks.

4. Method according to any of claims 1 to 3, wherein said switching from short-day light conditions to long-day light conditions is effected within 3 to 4 days after applying the 2-chloroethylphosphonic acid.

5. Method according to any of claims 1 to 4, wherein said winter simulation sub-period spans 3 to 8 weeks, preferably about 5 weeks, and preferably at temperatures between +2 and +12 degrees Celsius.

6. Method according to any of claims 1 to 5, wherein the 2-chloroethylphosphonic acid is applied as a solution with a concentration of 0.2 ‰ to 5 ‰, preferably as a spray, or as soil drench.

7. Method according to any of claims 1 to 6, wherein at least the generative growth takes place in a greenhouse.

8. Campanula obtained by the method as defined in claims 1 to 8.

9. Campanula Portenschlagiana having an upright spreading habit.

10. Campanula Portenschlagiana according to claim 9, which has branched stems (2).
